# EUROPÄISCHE PATENTSCHRIFT

(11) **EP 1 782 745 B1**
(45) Veröffentlichungstag und Bekanntmachung des Hinweises auf die Patenterteilung: **05.08.2009**
(21) Anmeldenummer: 06123584.2
(22) Anmeldetag: 07.11.2006
(51) Int. Cl.: A61B 19/00

(54) **Gewebemarker mit einem vorgeformten, distal geschlitzten Draht**
Tissue marker comprising a preformed wire with a distal slit
Marqueur de tissu comprenant un fil préformé fendu distalement

(30) Priorität: 07.11.2005 DE 102005053464
(43) Veröffentlichungstag der Anmeldung: 09.05.2007
(73) Patentinhaber: SOMATEX Medical Technologies GmbH, 14513 Teltow (DE)
(72) Erfinder: Hornscheidt, Dirk, 12621, Berlin (DE); Kniep, Frank, 14979, Großbeeren (DE); Kilka, Mario, 15913, Goyatz (DE); Neumann, Chris, 13189, Berlin (DE)
(74) Vertreter: Eisenführ, Speiser & Partner

(56) Entgegenhaltungen:
- WO-A2-20/04012600
- US-A- 2 198 319
- US-A- 3 001 522
- US-A- 5 925 059
- US-A- 6 093 154
- US-B1- 6 371 904
- US-B1- 6 766 186

## Beschreibung

Die Erfindung betrifft ein chirurgisches Instrument, insbesondere ein Markerinstrument zum Markieren von Körpergewebeabschnitten. Das Instrument soll insbesondere zur Markierung von Tumorgewebe vor dem chirurgischen Entfernen dieses Gewebes geeignet sein.

Derartige Markierungsinstrumente sind grundsätzlich bekannt. So ist beispielsweise in der DE 44 24 394 A1 sowohl ein solches Instrument als auch dessen Verwendung beschrieben.

Ein Bestandteil des Instrumentes ist eine Hohlnadel zum Einstechen in Körpergewebe. Die Hohlnadel schließt ein Lumen ein, welches wenigstens an seinem distalen Ende und in der Regel auch an seinem proximalen Ende offen ist. In radialer Richtung ist das Lumen durch eine Innenwand der Hohlnadel begrenzt.

Weiterer Bestandteil des chirurgischen Instrumentes ist ein in dem Lumen geführtes, bezüglich der Hohlnadel längsverschiebliches Element. Dieses längsverschiebliche Element besitzt distale Endabschnitte, die eine derartige Vorbiegung aufweisen, dass sie im gestreckten Zustand elastisch vorgespannt sind und sich im entspannten Zustand krümmen. Die Anordnung des längsverschieblichen Elementes ist dabei derart, dass seine vorgebogenen distalen Endabschnitte wahlweise aus der offenen, distalen Mündung des Lumens herauszuschieben sind und sich dabei krümmen oder in das Lumen zurückzuziehen sind und dabei gestreckt werden.

Wie erwähnt, dient ein derartiges Instrument zum Markieren von Körpergewebe. Dazu wird zunächst die Hohlnadel mit in das Lumen zurückgezogenem Draht in das zu markierende Gewebe eingestochen. Anschließend wird der Draht in der Hohlnadel vorgeschoben, so dass die im Lumen gestreckten, vorgespannten, distalen Endabschnitte aus der offenen Mündung des Lumens distal aus der Hohlnadel heraustreten. Die Vorbiegung der distalen Endabschnitte führt dazu, dass diese sich beim Austritt aus der offenen Mündung des Lumens in die durch die Vorbiegung bestimmte Richtung krümmen und auf diese Weise in dem zu markierenden Gewebe verankern. Soll das chirurgische Instrument repositioniert oder entfernt werden, kann das längsverschiebliche Element auch in die Hohlnadel zurückgezogen werden. Dadurch werden die vorgebogenen, distalen Endabschnitte in dem Lumen wieder gestreckt. Der Durchmesser des Lumens der Hohlnadel ist nicht wesentlich größer als der Außendurchmesser des längsgestreckten Elementes in dessen gestreckten Zustand.

Zu den Anforderungen, die an ein derartiges Markierungsinstrument gestellt werden, zählen eine einfache und zuverlässige Handhabung sowie der Wunsch, ein Gewebevolumen definiert markieren zu können. Zu dem letztgenannten Zweck muss das Markierungsinstrument exakt positionierbar sein. Außerdem soll dass Markierungselement samt des längsverschieblichen Elementes möglichst repositionierbar sein. Gleichzeitig soll das längsverschiebliche Element mit Hilfe seiner vorgebogenen, distalen Endabschnitte eine möglichst große Rückhaltekraft im Gewebe bewirken. Das längsverschiebliche Element soll in der Hohlnadel möglichst leicht vorzuschieben sein. Der Außendurchmesser der Nadel soll möglichst < 1 mm. Und schließlich muss das Instrument auch als Einmalinstrument kostengünstig herzustellen sein. Ziel der Entwicklung eines entsprechenden Markierungselementes ist es daher, die verschiedenen, sich teilweise widersprechenden Eigenschaften so gut wie möglich zu realisieren und in Einklang miteinander zu bringen.

Bekannte Markierungsinstrumente wie dasjenige gemäß DE 44 24 394 lassen hinsichtlich der genannten Anforderungen Wünsche offen. So hat sich herausgestellt, dass die Herstellung nicht einfach und die Handhabung oft nicht völlig befriedigend ist.

Der Erfindung liegt daher die Aufgabe zugrunde, ein chirurgisches Instrument, insbesondere ein Markerinstrument zu schaffen, welches einen besseren Kompromiss bezüglich der vorgenannten Eigenschaften bietet.

Erfindungsgemäß wird diese Aufgabe durch ein chirurgisches Instrument der zuvor beschriebenen Art erfüllt, bei dem das längsverschiebliche Element von einem Draht mit einem Längsschlitz gebildet ist. Der Längsschlitz erstreckt sich von einem Fußpunkt an einem proximalen Schlitzende bis zu einem offenen distalen Schlitzende und trennt auf diese Weise die distalen Endabschnitte des längsverschieblichen Elementes voneinander. Anders als das bekannte Markierungselemente, gemäß DE 44 24 394, bei dem das längsverschiebliche Element von miteinander verdrillten Einzeldrähten gebildet ist, die jeweils einen vorgebogenen distalen Endabschnitt bilden, kann das längsverschiebliche Element von einem einzigen Draht gebildet, der in einem distalen Längsabschnitt in Längsrichtung geschlitzt ist, so dass das längsverschiebliche Element wenigstens in seinem distalen Bereich einstückig ist. US-676 6 186-B1 offenbart ein solches Markierungsinstrument, das die Merkmale aufweist, die im Oberbegriff des Anspruchs 1 zusammengefaßt sind.

Die Erfindung beruht auf der Erkenntnis, dass die Handhabungseigenschaften bekannter Markierungselemente sehr stark von Herstellungstoleranzen abhängen. Versuche mit einer Vielzahl unterschiedlicher Lösungen haben schließlich überraschend dazu geführt, dass das erfindungsgemäße Markierungsinstrument nicht nur weniger kritisch in bezug auf Fertigungstoleranzen ist, sondern auch im übrigen sehr gute Eigenschaften aufweist.

Als vorteilhaft hat es sich erwiesen, wenn die Vorbiegung der distalen Endabschnitte erst mit einem Abstand vom Fußpunkt des Längsschlitzes beginnt, so dass die distalen Endabschnitte im Bereich ihres Ursprungs neben dem proximalen Schlitzende ungebogen sind. Auf diese Weise können wirksam Ermüdungen des längsverschieblichen Elementes im Bereich des Fußpunktes des Längsschlitzes vermieden werden, so dass sich die Zuverlässigkeit des chirurgischen Instrumentes erhöht. Die Vorbiegung ist dabei von der Art, dass sich die distalen Endabschnitte beim Austritt aus der offenen Mündung des Lumens vom Längsschlitz und damit voneinander wegbiegen.

Der Längsschlitz verläuft vorzugsweise wenigstens über den größten Teil seiner Länge entlang der Längsachse des längsverschieblichen Elementes oder parallel zu ihr. Auf diese Weise ergeben sich distale Endabschnitte, die über den größten Teil ihrer Länge einen einheitlichen Querschnitt besitzen. Wenn der Längsschlitz entlang der Längsachse des längsverschieblichen Elementes verläuft und damit das längsverschiebliche Element wenigstens über den größten Teil seiner Länge mittig teilt, besitzen die distalen Endabschnitte über den größten Teil ihrer Länge eine identische Querschnittsform.

Wenn die Vorbiegung dieser distalen Endabschnitte voneinander abweicht, hat dies den Vorteil, dass dies eine Unterscheidung verschiedner, unterschiedlich orientierter längsverschieblicher Elemente erlaubt.

In einer bevorzugten Variante des chirurgischen Instrumentes sind die distalen Endabschnitte unterschiedlich lang. Dies wird vorzugsweise dadurch bewirkt, dass der Längsschlitz im Bereich seines distalen Schlitzendes schräg zur Längsachse des längsverschieblichen Elementes verläuft, so dass er in einem offenen Schlitzende im Umfang des längsverschieblichen Elementes endet und sich das Längere der distalen Endabschnitte des längsverschieblichen Elementes distal des offenen Schlitzendes fortsetzt. In dem distal des offenen Schlitzendes befindlichen Längsabschnitt des längeren im distalen Endes besitzt das längere distale Ende vorzugsweise einen Querschnitt, der dem Querschnitt des längsverschieblichen Elementes in einem Längsabschnitt proximal des Fußpunktes des Längsschnittes entspricht. Ein derartiges längsverschiebliches Element lässt sich nicht nur in vorteilhafter Weise dadurch herstellen, dass ein Draht einheitlichen Durchmessers mit einem entsprechenden Schlitz versehen wird. Ein chirurgisches Instrument mit einem solchen längsverschieblichen Element besitzt darüber hinaus auch vorteilhafte Eigenschaften hinsichtlich seiner Handhabung. Insbesondere kann der letzte, ungeschlitzte distale Endabschnitt des längeren distalen Endes derart angespitzt sein, dass er zusammen mit dem angespitzten Ende der Hohlnadel eine geschlossene Spitzengeometrie bilden, bei der das längsverschiebliche Element in seiner in die Hohlnadel zurückgezogenen Position mit seiner ungeschlitzten Spitze geringfügig aus dem distalen Ende der Hohlnadel herausragt. Dadurch wird die Spitze der Hohlnadel durch die Spitze des längsverschieblichen Elementes unterstützt und erlaubt im Ergebnis eine Hohlnadel mit geringerer Wandstärke und damit geringerem Außendurchmesser. Die Hohlnadel kann in diesem Fall sogar eine stumpfe Hohlnadel sein, das die Spitze des längsverschieblichen Elementes letztendlich die Spitze des chirurgischen Instrumentes beim Einstechen in Körpergewebe bildet. Auch kann die Hohlnadel aus Kunststoff bestehen und damit z.B. magnetresonanz-kompatibel sein.

In allen Ausführungsvarianten ist der Schlitz vorzugsweise durch Laserschneiden oder durch Drahterosion hergestellt. Die Drahterosion ist ein Funkenerosionsverfahren mit einem Draht als Gegenpol zu dem zu schlitzenden längsverschieblichen Element. Die Schlitzbreite beträgt vorzugsweise zwischen 0,03 und 0,1 mm. Der Außendurchmesser des längsverschieblichen Elementes beträgt vorzugsweise zwischen 0,4mm und 0,6mm und beeinflusst maßgeblich, die gewünschte, vom Arzt gespürte Steifigkeit beim Vorschieben des längsverschieblichen Elementes. Die Hohlnadel hat vorzugsweise einen Außendurchmesser von weniger als 1,2mm oder besser 1 mm.

Die distalen Endabschnitte des längsverschieblichen Elementes sind vorzugsweise angespitzt, um ein leichtes Eindringen der distalen Endabschnitte in das Körpergewebe zu ermöglichen, wenn das längsverschiebliche Element distal aus der offenen Mündung des Lumens herausgeschoben wird. Dabei hat es sich als vorteilhaft erwiesen, wenn die distalen Endabschnitte einen schrägen Anschnitt oder Anschliff besitzen. Die dadurch bewirkte schräge Anschliff- oder Anschnittfläche verläuft vorzugsweise in einem spitzen Winkel zu der Längsachse des jeweiligen distalen Endabschnittes und ist vorzugsweise so angeordnet, dass sie bei gestreckten distalen Endabschnitte jeweils nach innen weist.

In einer bevorzugten Ausführungsvariante besitzt das längsverschiebliche Element zwei Längschlitze, die in unterschiedlichen Längsebenen verlaufen und bewirken, dass das längsverschiebliche Element mehr als zwei distale Endabschnitte aufweist, in einer bevorzugten Ausführungsvariante beispielsweise vier distale Endabschnitte. Zu diesem Zweck verlaufen die Längsschlitze vorzugsweise in zueinander senkrecht stehenden Ebenen, die sich vorzugsweise auf der Längsachse des längsverschieblichen Elementes schneiden.

Das längsverschiebliche Element ist vorzugsweise ein Draht aus einem superelastischen Material, insbesondere einer superelastischen Metalllegierung wie Nitinol. Nitinol ist eine bekannte Nickel-Titan-Legierung, die superelastische Eigenschaften besitzt.

Die Hohlnadel kann in bevorzugten Ausführungsvarianten aus Titan, einer Titanlegierung oder aus einer biokompatiblen Stahllegierung bestehen. Letzteres führt dazu, dass das gesamte chirurgische Instrument für den Einsatz in Verbindung mit Magnetresonanztomographen geeignet ist (MR-kompatibel). Der Einsatz von Titan als Werkstoff für die Hohlnadel ist üblicherweise aufgrund der Rauhigkeit des Titans problematisch, da die Rauhigkeit das Hinausschieben des längsverschieblichen Elementes allzu sehr behindert. Bei längsverschieblichen Elementen der beanspruchten Art ist die Rauhigkeit des Titans indes unschädlich.

Alternative, vorteilhafte Materialien für die Hohlnadel sind MR-kompatible Kunststoffe, insbesondere Polyethteretherketone (PEEK) oder kohlefaserverstärkte Kunststoffe.

Die Oberfläche des längsverschieblichen Elementes ist vorzugsweise zum Beispiel durch Beizen aufgerauht und auf diese Weise unter Ultraschallbeobachtung besser sichtbar.

Die Vorbiegung der distalen Endabschnitte ist vorzugsweise derart, dass die distalen Endabschnitte im entspannten Zustand eine Krümmung von wenigstens 180° annehmen. Dies bewirkt eine sichere Verankerung des chirurgischen Instrumentes im zu markierenden Körpergewebe. Wie zuvor bereits erwähnt, kann die Vorbiegung der verschiedenen distalen Endabschnitte eines längsverschieblichen Elementes unterschiedlich sein, wodurch im chirurgischen Einsatz eine Unterscheidung verschiedener längsverschieblicher Elemente grundsätzlich identischer Geometrie anhand einer unterschiedlichen Orientierung ihrer distalen Endabschnitte im Gewebe möglich wird.

Es hat sich herausgestellt, dass eine derartige Vorbiegung auch nach mehrfachem Hinausschieben und wieder Zurückziehen des längsverschieblichen Elementes erhalten bleibt, wenn die distalen Endabschnitte zunächst mit einem engeren Radius und um einen größeren Winkel herum vorgebogen werden, als es der gewünschten dauerhaften Endgeometrie entspricht, und die distalen Endabschnitte anschließend einmal wieder gestreckt werden. Nach diesem Strecken nehmen die freien Enden eine Geometrie an, die sie dann über eine Vielzahl von Lastzyklen in Form eines Hinausschiebens und wieder Zurückziehens beibehalten.

Vorzugsweise wird das längsverschiebliche Element von einem Draht gebildet, dessen Gesamtlänge im gestreckten Zustand der vorgebogenen distalen Endabschnitte so bemessen, dass ein proximales Ende des Drahtes auch dann noch aus einem proximalen Ende der Hohlnadel herausragt, wenn die vorgebogenen, distalen Endabschnitte des Drahtes aus der distalen Mündung des Lumens der Hohlnadel hinausgeschoben sind. Am proximalen Ende des Drahtes sind vorzugsweise zwei Längsmarkierungen vorgesehen, die einen Längsabstand voneinander haben, welcher dem Maß entspricht, um das das längsverschiebliche Element von seiner vollständig in das Lumen zurückgezogenen Position bis zu einer Längsposition vorgeschoben ist, bei dem die vorgebogenen distalen Endabschnitte aus der offenen distalen Mündung des Lumens der Hohlnadel hinausgeschoben sind.

In einer bevorzugten Ausführungsvariante ist am proximalen Ende des chirurgischen Elementes ein Handgriff vorgesehen, der vorzugsweise lösbar mit dem proximalen Ende der Hohlnadel verbunden ist und der einen Schieber enthält, welcher lösbar mit dem proximalen Ende des längsverschieblichen Elementes verbunden ist. Der Schieber ist zum einen zwischen zwei Längspositionen hin und her beweglich und derart mit dem längsverschieblichen Element verbunden, dass das längsverschiebliche Element in einer Endposition des Schiebers vollständig in das Lumen der Hohlnadel zurückgezogen ist, während die zweite Endposition des Schiebers derjenigen Längsposition des längsverschieblichen Elementes entspricht, bei dem die vorgebogenen distalen Endabschnitte des längsverschieblichen Elementes vollständig aus der offenen distalen Mündung des Lumes der Hohlnadel hinausgeschoben sind. Ein derartiger Handgriff mit Schieber vereinfacht die Handhabung des chirurgischen Elementes, weil ein Benutzer nicht mehr auf Längsmarkierungen an dem längsverschieblichen Element achten muss, um die jeweilige richtige Längsposition des längsverschieblichen Elementes bezüglich der Hohlnadel einzustellen. Markierungen an dem längsverschieblichen Element werden somit überflüssig. Außerdem erleichtert der Griff das Vorschieben des Drahtes.

Anstelle eines Schiebers kann auch ein anderes Schiebeelement vorgesehen sein, welches beispielsweise nach Art eines Kugelschreibers durch einen Druckmechanismus zwischen zwei Längspositionen hin- und her zu bewegen ist.

In einer bevorzugten Ausführungsvariante kann der Schieber in eine dritte Position gebracht werden, in der der Schieber von dem längsverschieblichen Element getrennt ist, so dass die Hohlnadel mit Hilfe des Griffes unabhängig von dem längsverschieblichen Element zu bewegen ist. Letzteres ist zum Beispiel erforderlich, wenn das längsverschiebliche Element an einem gewünschten Ort im Körpergewebe eines Patienten positioniert und durch Vorschieben der vorgebogenen distalen Endabschnitte fixiert ist und die Hohlnadel entfernt werden soll, ohne gleichzeitig das längsverschiebliche Element mit zu entfernen. Schließlich soll das längsverschiebliche Element als Markierungselement an der vorgesehenen Position im Körpergewebe des Patienten verbleiben.

Der Griff und der Schieber an dem Griff sind in bezug auf die letztgenannte Ausführungsvariante vorzugsweise derart gestaltet, dass ein Benutzer beim Bewegen des Schiebers von der ersten oder zweiten Längsposition in die dritte Position einen spürbaren Widerstand überwinden muss. Auf diese Weise kann ein Benutzer den Schieber praktisch ohne Widerstand zwischen der ersten und zweiten Längsposition hin und her bewegen, um das längsverschiebliche Element aus der offenen distalen Mündung der Hohlnadel hinaus zu schieben oder das längsverschiebliche Element vollständig in die Hohlnadel zurückzuziehen. Soll dann schließlich das längsverschiebliche Element losgelassen werden, muss der Benutzer den Schieber über einen spürbaren Widerstand hinwegbewegen, um dies zu bewirken.

In einer bevorzugten Ausführungsvariante ist einer Rastung vorgesehen, dass der Schieber jeweils in der ersten bzw. der zweiten Endposition einrastet, so dass der längsverschiebliche Draht seine jeweilige erste oder zweite Längsposition beibehält.

Schließlich ist es vorteilhaft, wenn der Schieber in eine vierte Position zu bewegen ist und in dieser Position bewirkt, dass der Griff von der Hohlnadel getrennt ist.

Unabhängig von der konkreten Gestaltung des längsverschieblichen Elementes kann der Griff beispielsweise auch im Zusammenhang mit solchen längsverschieblichen Elementen verwendet werden, die von zwei miteinander verdrillten Drähten gebildet sind.

Um das Positionieren und insbesondere das Repositionieren des längsverschieblichen Elementes im Körpergewebe eines Patienten zu erleichtern, kann das längsverschiebliche Element mit einer Hülle versehen sein, die zusammen mit dem längsverschieblichen Element aus dem Lumen der Hohlnadel hinauszuschieben oder in dieses zurückzuziehen ist. Die Hülle hat die Aufgabe, die vorgebogenen, distalen Endabschnitte des längsverschieblichen Elementes zunächst beieinander zu halten, so dass sie nicht ihre vorgekrümmte Form annehmen, sobald sie aus der offenen distalen Mündung des Lumens hinausgeschoben werden. Dies erleichtert es, das längsverschiebliche Element zusammen mit der Hülle auch ohne Hohlnadel aus Körpergewebe zurückzuziehen, um das längsverschiebliche Element zu repositionieren. Erst wenn das längsverschiebliche Element schließlich an seine endgültige Position gebracht ist, kann auch die Hülle bezüglich des längsverschieblichen Elementes zurückgezogen werden, so dass sich die vorgebogenen distalen Endabschnitte des längsverschieblichen Elementes in der vorgesehenen Weise krümmen und im Körpergewebe verankern.

In einer alternativen Ausführungsvariante ist das längsverschiebliche Element ein Implantat, welches vollständig aus dem Lumen herausgeschoben werden kann. Ein solches Implantat kann - nachdem es einmal aus dem Lumen herausgeschoben ist - in der Regel nicht wieder in das Lumen zurückgezogen werden, so dass nach Abgabe des Implantates ein Repositionieren des Implantates in der Regel nicht mehr möglich ist. Es kann auch eine lösbare Kupplung zwischen einem jeweiligen Implantat und beispielsweise einem nachfolgend erwähnten Schieberdraht vorgesehen sein, die das Zurückziehen und damit das Repositionieren erlaubt.

Um ein Implantat der letztgenannten Art aus dem Lumen des chirurgischen Instruments herauszuschieben, ist vorzugsweise ein Schieberdraht vorgesehen, der proximal des längsverschieblichen Elementes ebenfalls längsverschieblich in dem Lumen geführt und derart ausgebildet ist, dass das längsverschiebliche Element als Implantat mittels des Schieberdrahtes wenigstens so weit aus der Mündung des Lumens herauszuschieben ist, dass das Implantat sich mit seinen vorgebogenen, distalen Endabschnitte derart im Gewebe verkrallen kann, dass das Implantat beim Zurückziehen der Hohlnadel im Gewebe verbleibt.

An Stelle eines einzigen Implantates können auch mehrere Implantate in Längsrichtung des Lumens magazinartig hintereinander angeordnet sein, die mittels des Schieberdrahtes einzeln aus dem Lumen herausgeschoben werden können. Dazu kann der Schieberdraht Längsmarkierungen besitzen, die in Längsrichtung des Schieberdrahtes einen Abstand voneinander haben, der der gestreckten Länge eines jeweiligen einzelnen Implantates in dem Lumen entspricht. Dies erlaubt es beispielsweise einem Arzt, den Schieberdraht jeweils genau so weit vorzugschieben, wie es erforderlich ist, um jeweils ein Implantat abzugeben.

Die Innenwand der Hohlnadel kann mindestens abschnittsweise mit einer Gleitbeschichtung versehen sein, die ein biokompatibles organisches Gleitmittel enthält. Ein besonders geeignetes Gleitmittel ist ein langkettiges Wachs. Das Gleitmittel hat vorzugsweise einen Schmelzpunkt von über 50°C.

Es ist allerdings ein Vorteil des längsverschieblichen Elementes in seiner erfindungsgemäßen Gestalt, dass eine Gleitbeschichtung deshalb nahezu überflüssig ist, weil die Reibung zwischen längsverschieblichem Element und Hohlnadel ohnehin gering ist.

Falls dennoch eine Gleitbeschichtung vorgesehen ist, ist es vorteilhaft diese deshalb auf der Innenwand der Hohlnadel angeordnet ist und nicht etwa außen auf dem längsverschieblichen Element weil die erste Variante zwei Vorteile mit sich bringt: Zum einen kann so der Eintrag von Gleitmittel ins Körpergewebe minimiert werden, zum anderen ist auf diese Weise sichergestellt, dass über die gesamte Verschiebelänge des längsverschieblichen Elementes genügend Gleitmittel zur Verfügung steht. Würde nämlich das Gleitmittel auf der Außenseite des längsverschieblichen Elementes angeordnet sein, könnte das Gleitmittel beim Vorschieben des längsverschieblichen Elementes sukzessive an den Partien abgetragen werden, mit denen das längsverschiebliche Element die Innenwand der Hohlnadel berührt.

Weitere vorteilhafte Ausführungsvarianten ergeben sich aus den hier nicht kommentierten Unteransprüchen sowie aus der nachfolgenden Beschreibung.

Die Erfindung soll nun anhand eines Ausführungsbeispiels mit Bezug auf die Figuren näher erläutert werden. Von den Figuren zeigt:
Figur 1: die wesentlichen Bestandteile einer einfachen Ausführungsform des erfindungsgemäßen chirurgischen Instrumentes;
Figur 2a bis g: das distale Ende des chirurgischen Elementes aus Figur 1 in verschiedenen Betriebszuständen;
Figur 3: eine Ansicht und eine Detailansicht einer ersten Ausführungsvariante eines längsverschieblichen Elementes des chirurgischen Elementes aus Figuren 1 und 2 mit längsgestreckten distalen Endabschnitten;
Figuren 4a und b: das längsverschiebliche Element aus Figur 3 mit entspannten distalen Endabschnitte in ihrer gebogenen Endgeometrie;
Figuren 5a bis d: das längsverschiebliche Element aus Figur 4 in zwei Herzstellungsschritten;
Figuren 6a und b: ein alternatives längsverschiebliches Element für ein chirurgisches Instrument ähnlich Figur 1, wobei die distalen Endabschnitte des längsverschieblichen Elementes im gestreckten Zustand dargestellt sind;
Figur 7: ein chirurgisches Instrument ähnlich Figur 1 mit einem Handgriff am proximalen Ende mit darin integrierten Schieber zum Bewegen des längsverschieblichen Elementes;
Figuren 8a bis c: ein chirurgisches Instrument, bei dem das längsverschiebliche Element eine auf Wunsch zu entfernende Hülle aufweist; und
Figur 9: ein chirurgisches Instrument, bei dem das längsverschiebliche Element die Form eines vollständig aus dem Lumen herauszuschiebenden Implantats hat.

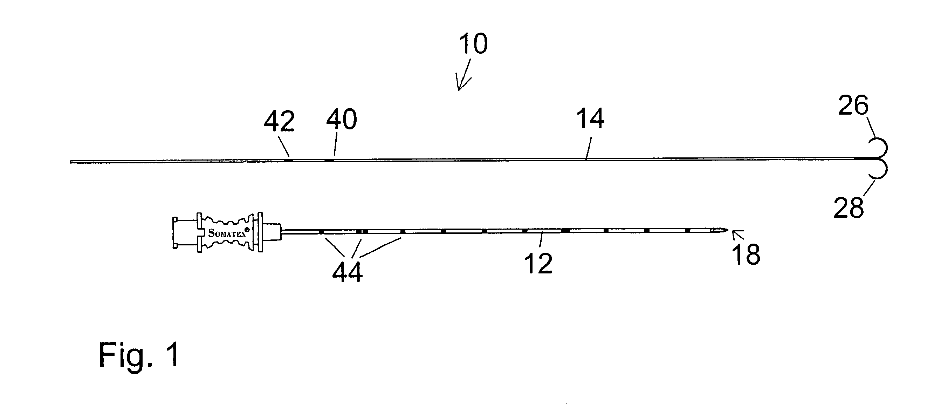

Wesentliche Bestandteile des chirurgischen Elementes 10 aus Figur 1 sind eine Hohlnadel 12 und ein längsverschieblicher Draht 14 als längsverschiebliches Element. Die Hohlnadel 12 besitzt ein Lumen 16, welches an beiden Längsenden offen ist und einen Innendurchmesser aufweist, der geringfügig größer ist, als ein Außendurchmesser des längsverschieblichen Drahtes 14. Ein distales Nadelende 18 der Hohlnadel 12 ist zum Einstechen in Körpergewebe angespitzt. Dort besitzt das Lumen 16 eine offene distale Mündung.

Der längsverschiebliche Draht 14 ist im Bereich seines distalen Endes 20 durch einen Schlitz 22 längs geschlitzt, so dass sich im dargestellten Ausführungsbeispiel zwei freie distale Endabschnitte 26 und 28 ergeben.

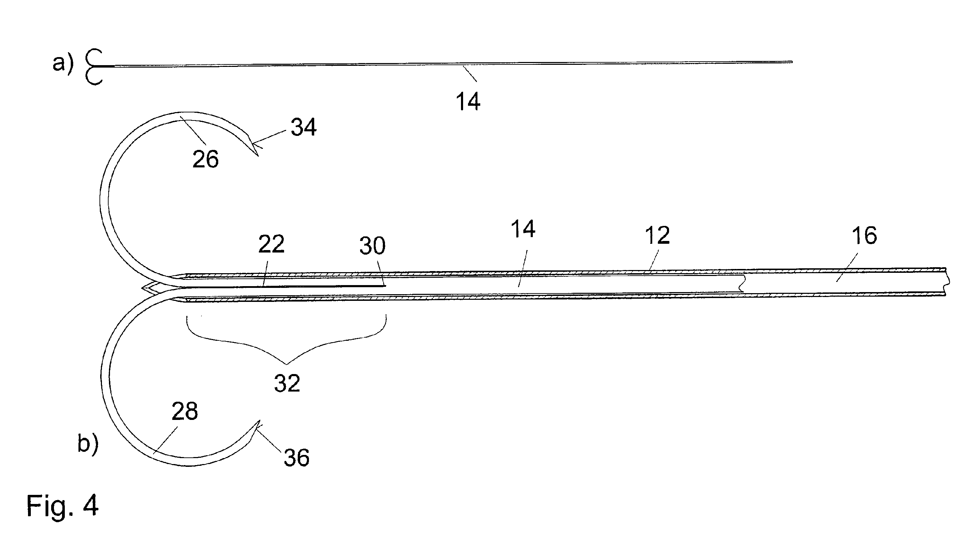

Wie den Figuren 4a und b, insbesondere Figur 4b zu entnehmen ist, sind die distalen Endabschnitte 26 und 28 um ein Kreisbogensegment von etwa 210° gebogen. Die Biegung der distalen Endabschnitte 26 und 28 setzt dabei nicht bereits im Fußpunkt 30 des Schlitzes 22 an. Vielmehr ist ein proximaler Längsabschnitt 32 der freien distalen Endabschnitte 26 und 28 ungebogen.

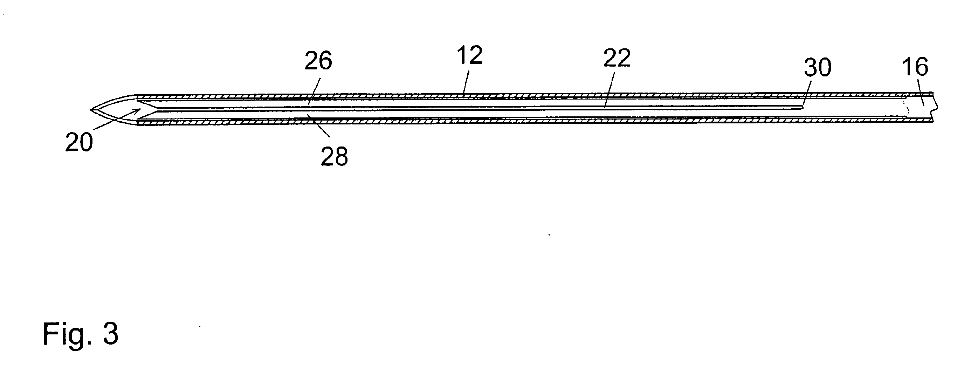

Die distalen Endabschnitte 26 und 28 sind angespitzt, und zwar durch einen Anschliff, der in einem spitzen Winkel von 20° zur Längsachse der distalen Endabschnitte 26 und 28 verläuft. Es entstehen auf diese Weise Schliffflächen 34 und 36, die im gestreckten Zustand der distalen Endabschnitte 26 und 28 (siehe Figur 3) einander zugewandt sind.

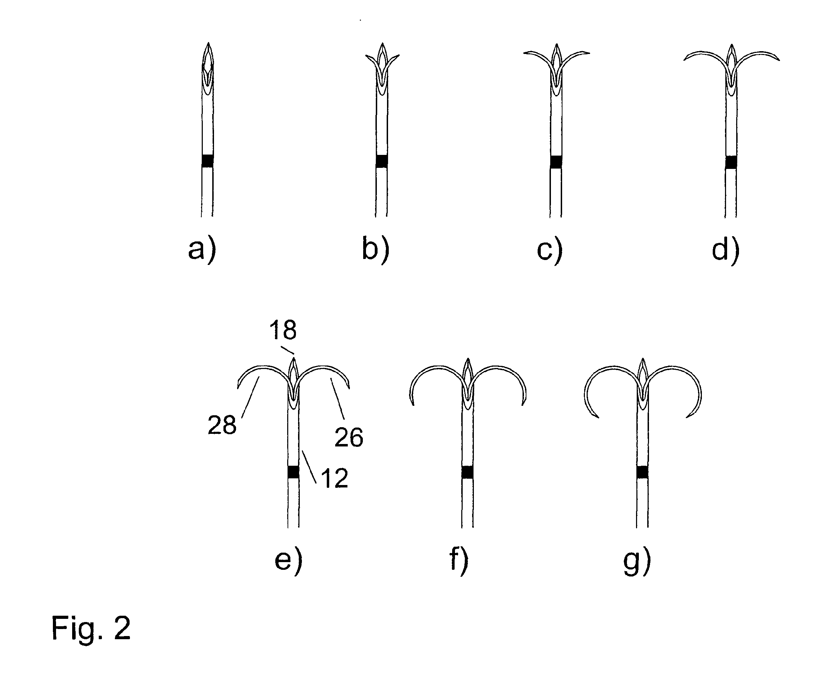

Wie den Figuren 2a bis g zu entnehmen ist, ist der längsverschiebliche Draht 14 beim Einstechen der Hohlnadel 12 in Körpergewebe zunächst so positioniert, dass seine distalen Endabschnitte 26 und 28 vollständig in das Lumen 16 der Hohlnadel zurückgezogen sind (Figur 2a). Wenn dann der längsverschiebliche Draht 14 in distale Richtung der Hohlnadel 12 vorgeschoben und damit die distalen Endabschnitte 26 und 28 aus der offenen distalen Mündung des Lumens 16 hinausgeschoben werden, krümmen sich die distalen Endabschnitte 26 und 28, wie in den Figuren 2b bis 2g dargestellt ist. In Figur 2g ist schließlich der Zustand dargestellt, in dem die vorgekrümmten Abschnitte der distalen Endabschnitte 26 und 28 vollständig aus dem Lumen 16 hinausgeschoben sind und ihre Endgeometrie im entspannten Zustand angenommen haben. Zwischen dem in Figur 2a dargestellten Zustand und dem in Figur 2g dargestellten Zustand ist der längsverschiebliche Draht 14 somit bezüglich der Hohlnadel 12 um eine Länge vorgeschoben worden, die der gestreckten Länge der gebogenen Abschnitte der distalen Endabschnitte 26 und 28 entspricht.

Der längsverschiebliche Draht 14 trägt zwei Markierungen 40 und 42, die einen Längsabstand voneinander haben, der genau der Länge entspricht, um die der längsverschiebliche Draht 14 zwischen dem in Figur 2a dargestellten Zustand und dem in Figur 2g dargestellten Zustand vorgeschoben ist. Die Markierungen 40 und 42 erlauben es somit dem Arzt festzustellen, dass der längsverschiebliche Draht 14 soweit aus der distalen offenen Mündung des Lumens 16 der Hohlnadel 12 hinausgeschoben ist, dass sich die vorgebogenen Abschnitte der distalen Endabschnitte 26 und 28 ganz außerhalb des Lumens 16 befinden.

Auch auf der Außenseite der Hohlnadel 12 sind Markierungen 44 angebracht, die es dem Arzt ermöglichen, die Einstichtiefe der Hohlnadel 12 in Körpergewebe zu erfassen.

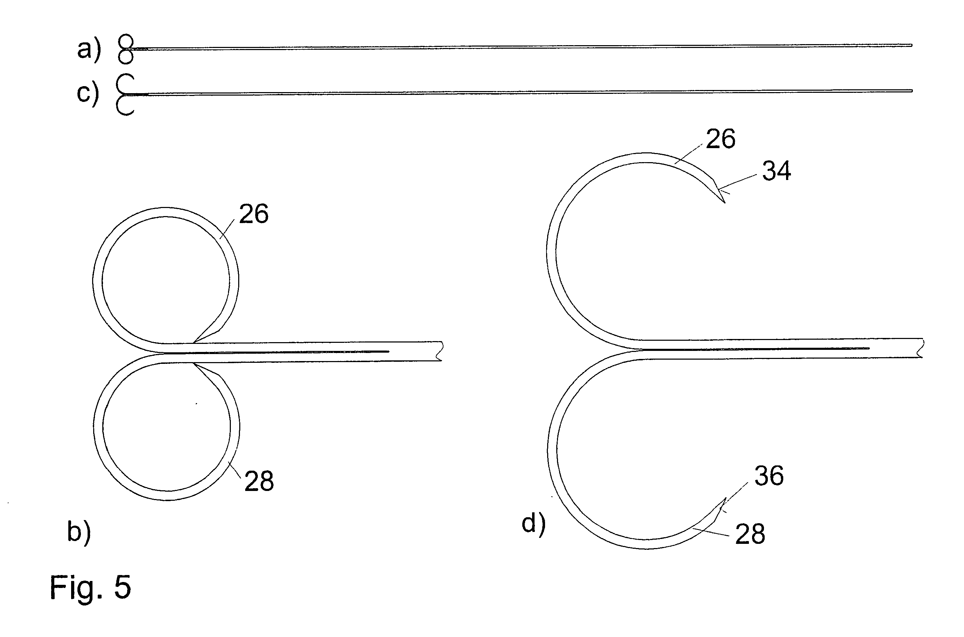

Um die in Figur 4 und auch Figur 5d abgebildete Endgeometrie der vorgebogenen Abschnitte der distalen Endabschnitte 26 und 28 zu erzeugen, werden die durch den Schlitz 22 voneinander getrennten Abschnitte zunächst um einen engeren Radius gekrümmt, als es der Endgeometrie entspricht. Die derart eng vorgekrümmten Abschnitte der distalen Endabschnitte 26 und 28 umschlingen dabei ein Kreiswinkelsegment von etwa 330° bis 340°; siehe Figur 5b. Anschließend werden die derart vorgebogenen Abschnitte der distalen Endabschnitte 26 und 28 gestreckt und nehmen anschließend die in Figuren 4b und 5d dargestellte Endgeometrie an, die die distalen Endabschnitte auch nach mehrfachen Strecken und anschließenden Entspannen (Krümmen) beibehalten.

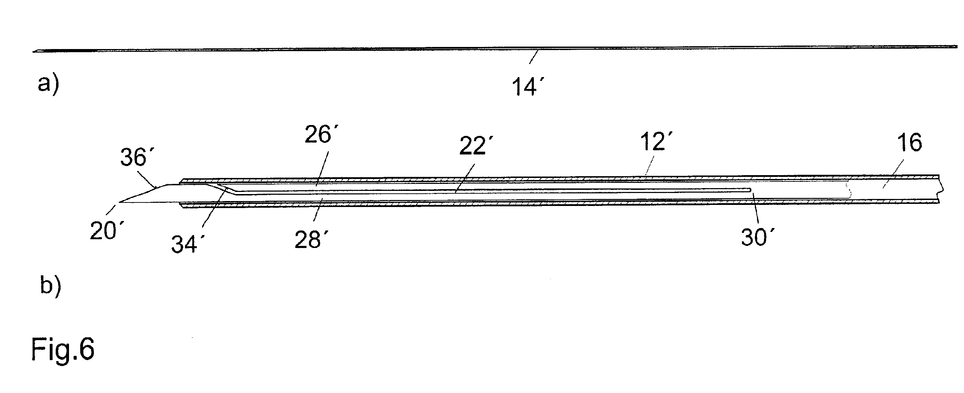

In Figur 6 ist ein alternativer längsgeschlitzter Draht 14' in gestrecktem Zustand dargestellt. Wie Figur 6 zu entnehmen ist, mündet der Schlitz 22' nicht ganz an der distalen Spitze des längsverschieblichen Drahtes 14', sondern mündet proximal des distalen Endes 20 seitlich im Umfang des längsverschieblichen Drahtes 14'. Auf diese Weise entstehen zwei unterschiedlich lange distale Endabschnitte 26' und 28' des längsverschieblichen Drahtes 14'.

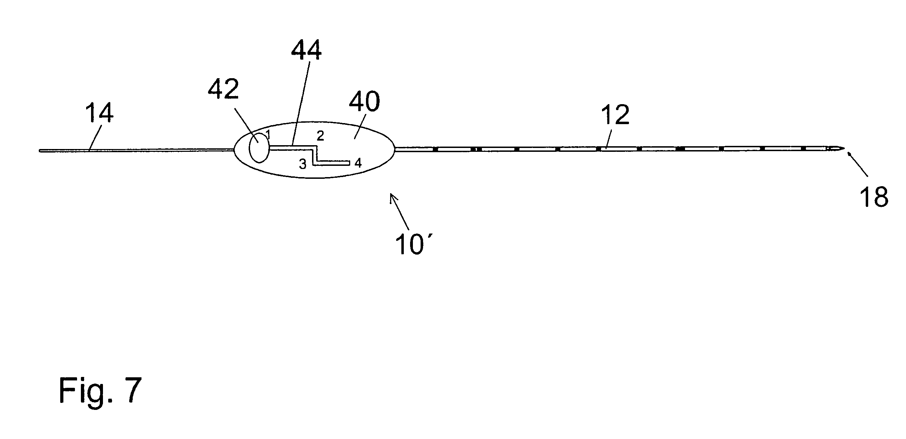

Figur 7 zeigt ein alternatives chirurgisches Instrument 10', bei welchem am proximalen Ende der Hohlnadel ein Griff 40 befestigt ist, der einen Schieber 42 aufweist, welcher als Schiebeelement mit dem längsverschieblichen Draht 14 verbunden ist. Mit Hilfe des als Schiebeelement dienenden Schiebers 42 kann der längsverschiebliche Draht 14 zwischen zwei Endpositionen in Längsrichtung der Hohlnadel verschoben werden. In der ersten Endposition, die auf dem Griff 40 mit "1" bezeichnet ist, ist das distale Ende des längsverschieblichen Drahtes 14 vollständig in die Hohlnadel 12 zurückgezogen. In dieser Position kann die Hohlnadel in Körpergewebe eingestochen werden. Wenn sich das distale Ende 18 der Hohlnadel 12 an der gewünschten Stelle im Körpergewebe befindet, kann der Schieber 42 in die zweite Endposition bewegt werden, die auf dem Griff 40 mit "2" gekennzeichnet ist. In dieser zweiten Endposition sind die gekrümmten Endabschnitte des längsverschieblichen Drahtes 14 vollständig aus der Mündung am distalen Ende 18 der Hohlnadel 12 hinausgeschoben.

Um ein versehentliches Betätigen des Schiebers 42 oder ein selbsttätiges Verschieben des längsverschieblichen Drahtes 14 zu vermeiden, rastet der Schieber 42 in den beiden Endpositionen "1" und "2" ein.

Wenn schließlich die gekrümmten, distalen Endabschnitte des längsverschieblichen Drahtes 14 an der gewünschten Stelle im Körpergewebe aus dem distalen Ende 18 aus der Hohlnadel 12 hinausgeschoben sind und der längsverschiebliche Draht 14 an der gewünschten Stelle verbleiben soll, kann der längsverschiebliche Draht 14 von dem Schiebeelement und damit von dem Schieber 42 getrennt werden, indem der Schieber 42 in die mit "3" bezeichnete Position auf dem Griff 40 bewegt wird. In Position "3" des Schiebers 42 sind der Handgriff 40, der Schieber 42 und auch die Hohlnadel 12 vom längsverschieblichen Draht 14 getrennt und können aus dem Körpergewebe zurückgezogen werden, während der längsverschiebliche Draht 14 im Körpergewebe verbleibt.

Schließlich ist in der dargestellten bevorzugten Ausführungsform des chirurgischen Instrumentes 10' auch eine vierte Position des Schiebers 42 vorgesehen, die auf dem Griff 40 mit "4" bezeichnet ist, in der der Griff 40 von der Hohlnadel 12 zu trennen ist. Damit ist es möglich, bei in Position gebrachtem längsverschieblichem Draht 14 den Griff 40 von dem längsverschieblichen Draht 14 und der Hohlnadel 12 zu trennen, so dass die Hohlnadel 12 bei Bedarf zusammen mit dem längsverschieblichen Draht 14 in Körpergewebe eingestochen bleiben kann, während der Griff 40 entfernt wird.

Wie Figur 7 zu entnehmen ist, ist für den Schieber 42 eine Kulissenführung 44 vorgesehen, die die Längsbewegung des Schiebers 42 zwischen den Endpositionen "1" und "2" des längsverschieblichen Drahtes 14 begrenzt und eine Seitwärtsbewegung des Schiebers 42 erfordert, um das mit dem Schieber 42 verbundene Schiebeelement von dem längsverschieblichen Draht 14 zu lösen. Die zum Lösen des längsverschieblichen Drahtes erforderliche Seitwärtsbewegung des Schiebers zwischen den Positionen "2" und "3" erfordert das Überwinden eines Widerstandes, so dass der Schieber 42 nicht versehentlich in die Position "3" bewegt werden kann. Auf diese Weise wird ein versehentliches Lösen des längsverschieblichen Drahtes 14 von dem Schieber 42 vermieden.

In einer nicht dargestellten, alternativen Ausführungsvariante ist der Schieber nur zwischen den Positionen "1" und "2" hin und her zu bewegen. Für das Lösen des Griffs von dem längsverschieblichen Draht oder von der Hohlnadel können ein oder zwei separate zusätzliche Betätigungselemente vorgesehen sein.

An anderer Stelle wurde bereits darauf hingewiesen, dass ein Griff 40 nicht auf die Verwendung mit einem längsverschieblichen Element 14 oder 14' der hier beanspruchten Art beschränkt ist, sondern auch mit anderen längsverschieblichen Elementen, beispielsweise in Form zweier oder mehrerer miteinander verdrillter Drähte einzusetzen ist. Der innere mechanische Aufbau des Griffes 40 ist von einem Fachmann auf verschiedene, ihm bekannte Art und Weise so zu verwirklichen, dass sich die zuvor beschriebene Funktionalität ergibt. Gemäß einer alternativen, hier nicht dargestellten Ausführungsvariante des Griffs weist dieser ein Druckelement nach Art eines Kugelschreibers auf, mit dem ein Schiebeelement zwischen den beiden Endpositionen für den längsverschieblichen Draht durch Druckbewegung hin und her zu bewegen ist.

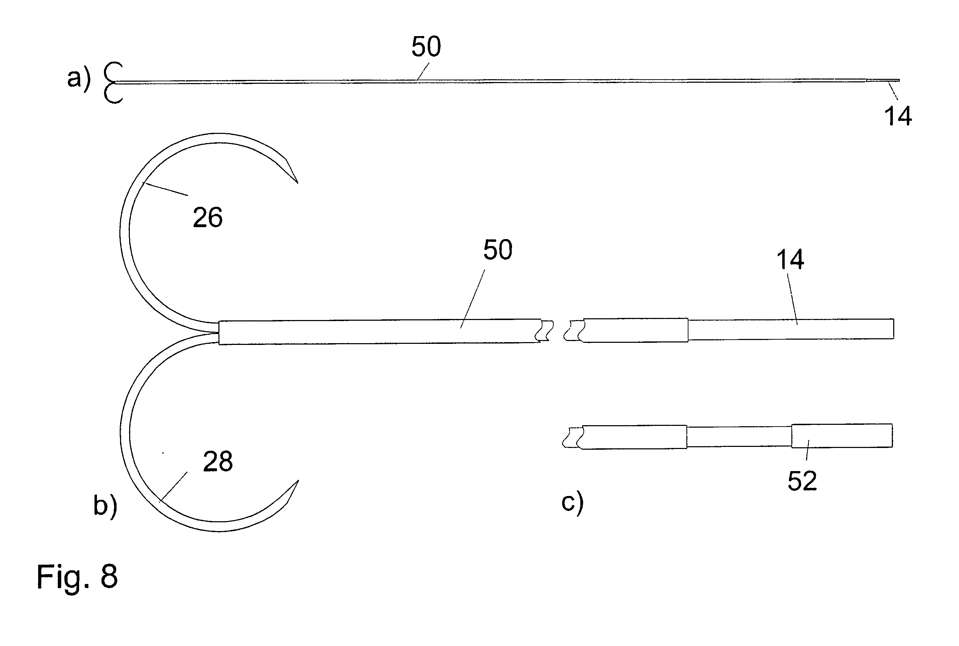

In Figur 8 ist ein längsverschieblicher Draht 14 nach Art der Figuren 1 bis 5 dargestellt, welcher von einer Hülle 50 umgeben ist. Die Hülle 50 ist gegenüber dem längsverschieblichen Draht 14 längsverschieblich. Die Hülle 50 hat die Aufgabe, in einer in Figur 8 nicht dargestellten Position die beiden vorgebogenen distalen Endabschnitte 26 und 28 im gestreckten Zustand zu halten, indem die Hülle die distalen Endabschnitte 26 und 28 über deren gesamte Länge umschließt. Der längsverschiebliche Draht 14 kann zusammen mit der bis zum distalen Ende 20 des längsverschieblichen Drahtes 14 vorgeschoben in Hülle 50 in das Lumen 16 der Hohlnadel 12 eingesetzt werden und zusammen mit der Hülle 50 distal aus der Mündung am distalen Ende 18 der Hohlnadel 12 hinausgeschoben werden. Die Hülle 50 verhindert dabei zunächst, dass sich die vorgebogenen distalen Endabschnitte 26 und 28 spreizen und krümmen. Erst wenn die Hülle 50 in Richtung des proximalen Endes des längsverschieblichen Drahtes 14 zurückgezogen wird, werden die distalen Endabschnitte 26 und 28 freigegeben und können sich krümmen.

Die Hülle braucht nur soweit zurückgezogen zu werden, wie dies in Figuren 8a und b dargestellt ist. Die Hülle 50 kann also nach dem Spreizen der distalen Endabschnitte 26 und 28 zusammen mit dem längsverschieblichen Draht 14 im Körpergewebe des Patienten verbleiben und erlaubt es, den längsverschieblichen Draht auch ohne Zuhilfenahme beispielsweise einer Hohlnadel wieder aus dem Körpergewebe zu entfernen, indem die Hülle 50 festgehalten wird und der längsverschiebliche Draht 14 bezüglich der Hülle zurückgezogen wird. Indem dabei die distalen Endabschnitte 26 und 28 in die Hülle 50 zurückgezogen werden und sich dadurch strecken wird verhindert, dass unnötig Körpergewebe beim Entfernen des längsverschieblichen Drahtes 14 verletzt wird. Wenn die distalen Endabschnitte 26 und 28 vollständig in die Hülle 50 zurückgezogen und damit gestreckt sind, kann die Hülle 50 zusammen mit dem längsverschieblichen Draht 14 aus dem Körpergewebe ohne weitere Verletzungsgefahr herausgezogen werden.

Wie in Figur 8c dargestellt ist, kann im proximalen Ende des längsverschieblichen Drahtes 14 ein Anschlagelement 52 fest mit dem längsverschieblichen Draht 14 verbunden sein, welches den Verschiebeweg des längsverschieblichen Drahtes 14 bezüglich der Hülle 50 in distaler Richtung begrenzt, so dass zwar wenigstens die distalen Endabschnitte 26 und 28 vollständig aus dem distalen Ende der Hülle 50 hinauszuschieben sind, die Hülle 50 aber nicht versehentlich über das proximale Ende des längsverschieblichen Drahtes 14 abgezogen wird.

Es sei an dieser Stelle darauf hingewiesen, dass die Hülle 50 in Kombination mit einem längsverschieblichen Draht 14' der Art, wie er in Figur 6 dargestellt ist, die Funktion der Hohlnadel 12 übernehmen und diese damit ersetzen kann. In diesem Sinne ist auch eine Hülle eine Ausführungsvariante einer Hohlnadel im Sinne Erfindung. Wie bereits an anderer Stelle erläutert, ist die Spitze am distalen Ende 20' des längsverschieblichen Drahtes 14' geeignet, ein Einstechen des chirurgischen Elementes in Körpergewebe zu erlauben, ohne dass es dazu einer angespitzten Hohlnadel bedarf.

Die Hülle 50 besteht vorzugsweise aus ausreichend steifen Kunststoff. Sie kann aber auch von einer dünnen Metallröhre gebildet sein.

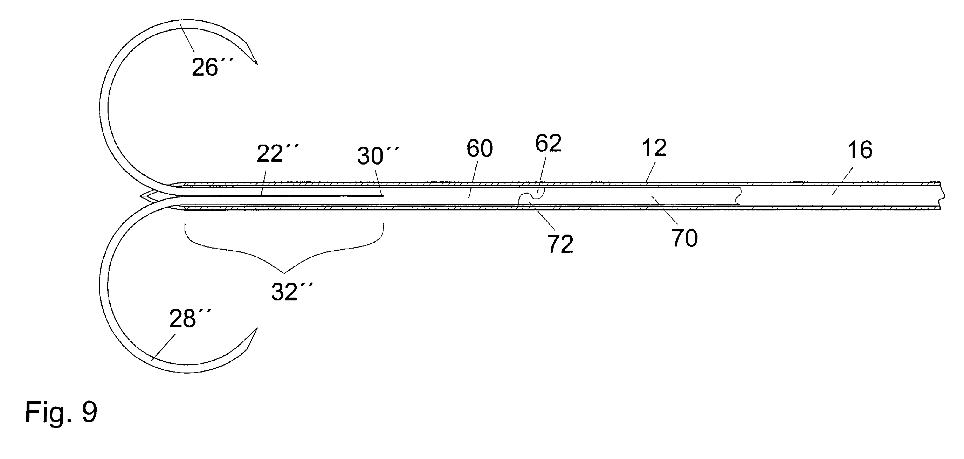

In Figur 9 ist ein chirurgisches Instrument abgebildet, bei dem ein längsverschiebliches Element 60 anders als bei den zuvor dargestellten Ausführungsvarianten vollständig aus der offenen Mündung am distalen Ende des Lumens 16 einer Hohlnadel hinauszuschieben ist. Diesem Zweck dient ein Schieberdraht 70, der proximal an das längsverschiebliche Element 60 angrenzt. Ein distales Ende des Schieberdrahtes 70 und das proximale Ende des längsverschieblichen Elementes 60 berühren sich im Bereich einer lösbaren Kupplung 62, 72, die von einer vorspringenden Nase 62 am proximalen Ende des längsverschieblichen Elementes 60 gebildet wird, die in Bezug auf die Längsrichtung des längsverschieblichen Elementes 60 eine entsprechende Nase 72 am distalen Ende des Schieberdrahtes 70 hintergreift. Solange sich die von den beiden Nasen 62 und 72 gebildete Kupplung zwischen längsverschieblichen Element 60 und Schieberdraht 70 im Lumen 16 befindet, kann das längsverschiebliche Element 60 mit Hilfe des Schieberdraht 70 auch dann noch in das Lumen 16 zurückgezogen werden, wenn die vorgebogenen distalen Endabschnitte 26" und 28" des längsverschieblichen Elementes 60 wie in dem in Figur 9 dargestellten Zustand bereits aus der offenen Mündung am distalen Ende des Lumens 16 hinausgeschoben sind.

Das längsverschiebliche Element 60 kann vollständig implantiert werden, indem es vollständig, einschließlich seiner Nase 62 am proximalen Ende aus dem Lumen 16 hinausgeschoben wird. Es ist offensichtlich, dass dann die von den beiden Nasen 62 und 72 gebildete Kupplung nicht mehr greift und ein Zurückziehen des längsverschieblichen Elementes 60 dann nicht mehr möglich ist.

In bezug auf die übrigen Merkmale entspricht das längsverschiebliche Element 60 dem längsverschieblichen Element 14 aus den Figuren 1 bis 5.

Der Schieberdraht 70 kann wie das längsverschiebliche Element 60 aus Titan oder einer Titanlegierung wie Nitinol bestehen. Alternativ ist auch eine biokompatible Stahllegierung ein geeignetes Material für den Schieberdraht 70.

## Patentansprüche

1. Chirurgisches Instrument, insbesondere Markierungsinstrument, zum Markieren von Körpergewebe, insbesondere von Tumorgewebe,
mit einer Hohlnadel (12) zum Einstechen in Körpergewebe,
welche ein von einer Innenwand der Hohlnadel radial begrenztes Lumen (16) einschließt, welches am distalen Nadelende (18) eine offene Mündung besitzt und
mit mindestens einem in dem Lumen geführten, längsverschieblichen Element (14; 14'; 60),
welches von einem Draht mit einem Längsschlitz (22; 22'; 22") gebildet ist, wobei der Längs- schlitz (22; 22': 22") sich von einem Fußpunkt (30; 30'; 30") an seinem proximalen Schlitzende bis zu einem offenen distalen Schlitzende erstreckt und distale Endabschnitte (26, 28; 26', 28'; 26", 28") des längsver- schieblichen Elementes (14; 14'; 60) voneinander trennt
wobei die distalen Endabschnitte (26; 28; 26', 28', 26", 28") eine derartige Vorbiegung aufweisen, dass die distalen Endabschnitte im gestreckten Zustand elastisch vorgespannt sind und im entspannten Zustand gekrümmt sind, wobei die Vorbiegung von dem Längsschlitz (22; 22'; 22") weg weist,
wobei die distalen Endabschnitte (26; 28; 26', 28'; 26", 28") des längsverschieblichen Elementes (14; 14'; 60) ausgehend von einer in das Lumen zurückgezogenen Position, in der die distalen Endabschnitte gestreckt sind, aus der offenen distalen Mündung des Lumens (16) hinauszuschieben sind und sich dabei krümmen,
**dadurch gekennzeichnet, dass** die Vorbiegung der distalen Endabschnitte (26, 28; 26', 28'; 26", 28") erst mit einem Abstand (32) in distaler Richtung vom Fußpunkt (30; 30'; 30") beginnt, so dass Ermüdungen des längsverschieblichen Elements im Bereich des Fußpunktes des Längsschlitzes beim Positionieren und Repositionieren vermieden werden.

2. Chirurgisches Instrument nach Anspruch 1, **dadurch gekennzeichnet, dass** der Längsschlitz (22; 22'; 22") wenigstens über den größten Teil seiner Länge entlang der oder parallel zur Längsachse des längsverschieblichen Elementes (14; 14'; 60) verläuft.

3. Chirurgisches Instrument nach einem der Ansprüche 1 oder 2, **dadurch gekennzeichnet, dass** die distalen Endabschnitte (26', 28') des längsverschieblichen Elementes (14') eine unterschiedliche Länge besitzen.

4. Chirurgisches Instrument nach Anspruch 2 und 3, **dadurch gekennzeichnet, dass** ein distaler Schlitzabschnitt des Längsschlitzes (22') schräg zur Längsachse des längsverschieblichen Elementes (14') verläuft, so dass sich das distale Schlitzende seitlich im Umfang des gestreckten, längsverschieblichen Elementes (14') befindet und der Längsschlitz auf dieses Weise ein kürzerer distaler Endabschnitt (26') des längsverschieblichen Elementes und ein längerer distaler Endabschnitt (28') des längsverschieblichen Elementes (14') voneinander trennt.

5. Chirurgisches Instrument nach Anspruch 4, **dadurch gekennzeichnet, dass** die Querschnittsfläche des längeren distalen Endabschnittes (28') in an den Längsschlitz seitlich angrenzenden Abschnitten geringer ist, als in unmittelbar distal des distalen Schlitzendes befindlichen Abschnitten.

6. Chirurgisches Instrument nach Anspruch 5, **dadurch gekennzeichnet, dass** der längere distale Endabschnitt (28') in seinem unmittelbar distal des distalen Schlitzendes befindlichen, ungeschlitzten Abschnitt eine Querschnittsfläche besitzt, die der Querschnittsfläche des längsverschieblichen Elementes (14') in seinem ungeschlitzten Abschnitt unmittelbar proximal des Fußpunktes (30') des Längsschlitzes (22') entspricht.

7. Chirurgisches Instrument nach einem der Ansprüche 3 bis 6, **gekennzeichnet durch** eine Hohlnadel (12) aus Kunststoff.

8. Chirurgisches Instrument nach einem der Ansprüche 1 bis 6, **gekennzeichnet durch** eine Hohlnadel (12) aus Titan oder einem titanhaltigen Material.

9. Chirurgisches Instrument nach einem der Ansprüche 1 bis 8, **dadurch gekennzeichnet, dass** die distalen Endabschnitte (26, 28; 26', 28'; 26", 28") des längsverschieblichen Elementes (14) angespitzt sind.

10. Chirurgisches Instrument nach Anspruch 9, **dadurch gekennzeichnet, dass** die distalen Endabschnitte (26, 28; 26, 28'; 26", 28") einen schrägen Anschliff oder Anschnitt aufweisen.

11. Chirurgisches Instrument nach Anspruch 10, **dadurch gekennzeichnet, dass** ein jeweiliger distaler Endabschnitt (26, 28; 26', 28'; 26", 28") eine schräge Anschliff- oder Anschnittfläche (34, 36; 34', 36'; 34", 36") aufweist, die im spitzen Winkel zu einer Mittelachse des jeweiligen distalen Endabschnittes (26, 28; 26', 28'; 26", 28") verläuft.

12. Chirurgisches Instrument nach Anspruch 10 oder 11, **dadurch gekennzeichnet, dass** eine jeweilige Anschlifffläche (34; 36; 34', 36'; 34", 36") oder Anschnittfläche bei in das Lumen (16) zurückgezogenem längsverschiebliche Draht (14; 14'; 60) nach innen weisend angeordnet ist.

13. Chirurgisches Instrument nach einem der Ansprüche 1 bis 12, **dadurch gekennzeichnet, dass** das längsverschiebliche Element zwei Längsschlitze aufweist, die in unterschiedlichen Längsebenen verlaufen, so dass sich vier distale Endabschnitte ergeben.

14. Chirurgisches Instrument nach einem der Ansprüche 1 bis 13, **dadurch gekennzeichnet, dass** das längsverschiebliche Element (14;14'; 60) von einem Draht aus einem superelastischen Material, insbesondere einer superelastischen Metalllegierung wie Nitinol gebildet ist.

15. Chirurgisches Instrument nach einem der Ansprüche 1 bis 14, **dadurch gekennzeichnet, dass** das längsverschiebliche Element die Form eines vollständig aus dem Lumen herauszuschiebenden Implantats (60) hat.

16. Chirurgisches Instrument nach Anspruch 15, **dadurch gekennzeichnet, dass** das chirurgische Instrument einen Schieberdraht (70) aufweist, der proximal des längsverschieblichen Elementes (60) längsverschieblich in dem Lumen geführt und derart ausgebildet ist, dass das längssverschiebliche Element (60) als Implantat mittels des Schieberdrahtes (70) weit genug aus der distalen Mündung des Lumens herauszuschieben ist, um zu bewirken, dass sich das Implantat ausreichend im zu markierenden Körpergewebe verkrallen kann, so dass das Implantat im Körpergewebe verbleibt, wenn die Hohlnadel samt Schieberdraht zurückgezogen wird.

17. Chirurgisches Instrument nach einem der Ansprüche 1 bis 16, **dadurch gekennzeichnet, dass** an einem proximalen Ende der Hohlnadel (12) ein Griff befestigt ist, der ein Schiebeelement aufweist, welches lösbar mit dem längsverschieblichen Element verbunden und zwischen zwei Längspositionen hin und her beweglich ist, von den eine erste Längsposition einem Zustand des chirurgischen Elementes entspricht, in dem die distalen Endabschnitte (26, 28; 26', 28') oder wenigstens ein kürzeres (26') der distalen Endabschnitte vollständig in das Lumen (16) der Hohlnadel (12) zurückgezogen sind oder ist, während eine zweite Längsposition einem Zustand des chirurgischen Elementes entspricht, in dem die wenigstens die gekrümmten Längsabschnitte der distalen Endabschnitte (26, 28; 26', 28') aus der offen Mündung am distalen Ende des Lumens (16) herausragen.

18. Chirurgisches Instrument nach Anspruch 17, **dadurch gekennzeichnet, dass** das Schiebeelement in ein dritte Position beweglich ist, in der das Schiebelement und damit der Griff von dem längsverschieblichen Element (14; 14') gelöst sind.

19. Chirurgisches Instrument nach einem der Ansprüche 1 bis 18, **dadurch gekennzeichnet, dass** das längsverschiebliche Element von einer bezüglich des längsverschieblichen Elementes längsverschieblichen Hülle umgeben ist, die zusammen mit dem längsverschieblichen Element aus dem Lumen (16) der Hohlnadel (12) hinauszuschieben ist.

20. Chirurgisches Instrument nach Anspruch 19, **dadurch gekennzeichnet, dass** die Hülle aus Kunststoff besteht.

## Claims

1. A surgical instrument, in particular a marking instrument, for marking body tissue, in particular tumour tissue, comprising
a hollow needle (12) for insertion into body tissue
which encloses a lumen (16) which is radially delimited by an inside wall of the hollow needle and which has an open mouth at the distal needle end (18), and
at least one longitudinally displaceable element (14; 14'; 60) which is guided in the lumen
and which is formed by a wire having a longitudinal slit (22; 22'; 22") wherein the longitudinal slit (22; 22'; 22") extends from a base point (30; 30'; 30") at its proximal slit end to an open distal slit end and separates distal end portions (26, 28; 26', 28'; 26", 28") of the longitudinally displaceable element (14; 14'; 60) from each other,
wherein the distal end portions (26; 28; 26', 28', 26", 28") have a pre-bend such that the distal end portions are elastically prestressed in the straightened condition and are curved in the relaxed condition, wherein the pre-bend faces away from the longitudinal slit (22; 22'; 22"),
wherein the distal end portions (26; 28; 26', 28', 26", 28") of the longitudinally displaceable element (14; 14'; 60) are to be pushed starting from a position of being retracted into the lumen, in which the distal end portions are straightened, out of the open distal mouth of the lumen (16) and in so doing curve,
**characterized in that** the pre-bend of the distal end portions (26, 28; 26', 28'; 26", 28") begins only at a spacing (32) in the distal direction from the base point (30; 30'; 30") so that fatigue phenomena of the longitudinally displaceable element in the region of the base point of the longitudinal slit in positioning and repositioning are avoided.

2. A surgical instrument according to claim 1 **characterized in that** the longitudinal slit (22; 22'; 22") extends at least over the greatest part of its length along or parallel to the longitudinal axis of the longitudinally displaceable element (14; 14'; 60).

3. A surgical instrument according to one of claims 1 and 2 **characterized in that** the distal end portions (26', 28') of the longitudinally displaceable element (14') are of a different length.

4. A surgical instrument according to claim 2, and claim 3 **characterized in that** a distal slit portion of the longitudinal slit (22') extends inclinedly relative to the longitudinal axis of the longitudinally displaceable element (14') so that the distal slit end is disposed laterally in the circumference of the straightened longitudinally displaceable element (14') and **in that** way the longitudinal slit separates a shorter distal end portion (26') of the longitudinally displaceable element and a longer distal end portion (28') of the longitudinally displaceable element (14') from each other.

5. A surgical instrument according to claim 4 **characterized in that** the cross-sectional area of the longer distal end portion (28'), in portions laterally adjoining the longitudinal slit, is smaller than in portions disposed immediately distally of the distal slit end.

6. A surgical instrument according to claim 5 **characterized in that**, in its unslitted portion disposed immediately distally of the distal slit end, the longer distal end portion (28') is of a cross-sectional area which corresponds to the cross-sectional area of the longitudinally displaceable element (14') in its unslitted portion immediately proximally of the base point (30') of the longitudinal slit (22').

7. A surgical instrument according to one of claims 3 to 6 **characterized by** a hollow needle (12) of plastic material.

8. A surgical instrument according to one of claims 1 to 6 **characterized by** a hollow needle (12) of titanium or a titanium-bearing material.

9. A surgical instrument according to one of claims 1 to 8 **characterized in that** the distal end portions (26, 28; 26', 28'; 26", 28") of the longitudinally displaceable element (14) are pointed.

10. A surgical instrument according to claim 9 **characterized in that** the distal end portions (26, 28; 26', 28'; 26", 28") have an inclined grind or chamfer.

11. A surgical instrument according to claim 10 **characterized in that** a respective distal end portion (26, 28; 26', 28'; 26", 28") has an inclined ground or chamfer surface (34, 36; 34', 36'; 34", 36") which extends at an acute angle relative to a centre line of a respective distal end portion (26, 28; 26', 28'; 26", 28").

12. A surgical instrument according to claim 10 or claim 11 **characterized in that** a respective ground surface (34, 36; 34', 36'; 34", 36") or chamfer surface is arranged facing inwardly when the longitudinally displaceable wire (14; 14'; 60) is retracted into the lumen (16).

13. A surgical instrument according to one of claims 1 to 12 **characterized in that** the longitudinally displaceable element has two longitudinal slits which extend in different longitudinal planes, thereby providing four distal end portions.

14. A surgical instrument according to one of claims 1 to 13 **characterized in that** the longitudinally displaceable element (14; 14'; 60) is formed by a wire of a superelastic material, in particular a superelastic metal alloy such as Nitinol.

15. A surgical instrument according to one of claims 1 to 14 **characterized in that** the longitudinally displaceable element is in the form of an implant (60) which is to be pushed completely out of the lumen.

16. A surgical instrument according to claim 15 **characterized in that** the surgical instrument has a slider wire (70) which proximally of the longitudinally displaceable element (60) is longitudinally displaceably guided in the lumen and is so adapted that the longitudinally displaceable element (60) as an implant is to be pushed out of the distal mouth of the lumen by means of the slider wire (70) sufficiently far to cause the implant to be able to dig sufficiently in body tissue to be marked so that the implant remains in the body tissue when the hollow needle together with the slider wire is withdrawn.

17. A surgical instrument according to one of claims 1 to 16 **characterized in that** fixed to a proximal end of the hollow needle (12) is a handle having a sliding element which is releasable connected to the longitudinally displaceable element and which is reciprocatingly movable between two longitudinal positions of which a first longitudinal position corresponds to a condition of the surgical element in which the distal end portions (26, 28; 26', 28') or at least a shorter one (26') of the distal end portions are or is completely retracted into the lumen (16) of the hollow needle (12) while a second longitudinal position corresponds to a condition of the surgical element in which at least the curved longitudinal portions of the distal end portions (26, 28; 26', 28') project out of the open mouth at the distal end of the lumen (16).

18. A surgical instrument according to claim 17 **characterized in that** the sliding element is movable into a third position in which the sliding element and therewith the handle are released from the longitudinally displaceable element (14; 14').

19. A surgical instrument according to one of claims 1 to 18 **characterized in that** the longitudinally displaceable element is surrounded by a sheath which is longitudinally displaceable with respect to the longitudinally displaceable element and which is to be pushed out the lumen (16) of the hollow needle (12) together with the longitudinally displaceable element.

20. A surgical instrument according to claim 19 **characterized in that** the sheath comprises plastic material.

## Revendications

1. Instrument chirurgical, notamment instrument de marquage, destiné à marquer des tissus du corps, notamment des tissus tumoraux, ledit instrument chirurgical comportant
une aiguille creuse (12) qui est destinée à piquer des tissus du corps et qui inclut une lumière (16) limitée radialement par une paroi intérieure de l'aiguille creuse et possédant à son extrémité distale (18) un orifice ouvert et
au moins un élément (14 ; 14' ; 60) qui est déplaçable longitudinalement et guidé dans la lumière et qui est formé par un fil doté d'une fente longitudinale (22 ; 22' ; 22"), la fente longitudinale (22 ; 22' ; 22") s'étendant à son extrémité proximale depuis une base (30 ; 30' ; 30") jusqu'à son extrémité distale ouverte et séparant l'une de l'autre des portions d'extrémité distale (26, 28 ; 26', 28' ; 26", 28") de l'élément (14 ; 14' ; 60) déplaçable longitudinalement,
les portions d'extrémité distales (26, 28 ; 26', 28' ; 26", 28") comportant une précambrure telle que les portions d'extrémité distales sont précontraintes élastiquement à l'état allongé et sont incurvées à l'état relâché,
la précambrure étant dirigée depuis la fente longitudinale (22; 22'; 22"),
les portions d'extrémité distales (26, 28 ; 26', 28' ; 26", 28") de l'élément (14 ; 14' ; 60) déplaçable longitudinalement pouvant être sorties par l'orifice distal ouvert de la lumière (16) depuis une position, rétractée dans la lumière, dans laquelle les portions d'extrémité distales sont allongées,
**caractérisé en ce que** la précambrure des portions d'extrémité distales (26, 28 ; 26', 28' ; 26", 28') commence seulement à une distance (32) de la base (30 ; 30' ; 30") dans une direction distale de façon à éviter que l'élément déplaçable longitudinalement ne fatigue au niveau de la base de la fente longitudinale lors du positionnement et du repositionnement.

2. Instrument chirurgical selon la revendication 1, **caractérisé en ce que** la fente longitudinale (22 ; 22' ; 22") s'étend, au moins sur la plus grande partie de sa longueur, le long de l'axe longitudinal, ou parallèlement à celui-ci, de l'élément (14 ; 14' ; 60) déplaçable longitudinalement.

3. Instrument chirurgical selon l'une des revendications 1 ou 2, **caractérisé en ce que** les portions d'extrémité distales (26', 28') de l'élément (14) déplaçable longitudinalement ont des longueurs différentes.

4. Instrument chirurgical selon les revendications 2 et 3, **caractérisé en ce qu'**une portion distale de la fente longitudinale (22') s'étend obliquement par rapport à l'axe longitudinal de l'élément (14') déplaçable longitudinalement, de sorte que l'extrémité distale de la fente est placée latéralement sur l'étendue de l'élément (14) déplaçable longitudinalement, lorsqu'il est déployé, et la fente longitudinale sépare ainsi l'une de l'autre une portion d'extrémité distale (26'), la plus courte, de l'élément déplaçable longitudinalement et une portion d'extrémité distale (28'), la plus longue, de l'élément (14) déplaçable longitudinalement.

5. Instrument chirurgical selon la revendication 4, **caractérisé en ce que** la portion d'extrémité distale la plus longue (28') a une section qui est plus petite dans des portions bordant latéralement la fente longitudinale que dans des portions se trouvant tout près de l'extrémité distale de la fente.

6. Instrument chirurgical selon la revendication 5, **caractérisé en ce que** la portion d'extrémité distale la plus longue (28') présente, dans sa portion non fendue située tout près de l'extrémité distale de la fente, une section qui correspond à la section de l'élément (14) déplaçable longitudinalement dans sa portion non fendue tout près de l'extrémité proximale de la fente longitudinale (22) où se trouve la base (30').

7. Instrument chirurgical selon l'une des revendications 3 à 6, **caractérisé par** une aiguille creuse (12) en matière plastique.

8. Instrument chirurgical selon l'une des revendications 1 à 6, **caractérisé par** une aiguille creuse (12) en titane ou en matériau contenant du titane.

9. Instrument chirurgical selon l'une des revendications 1 à 8, **caractérisé en ce que** les portions d'extrémité distales (26, 28 ; 26', 28' ; 26", 28") de l'élément (14) déplaçable longitudinalement sont appointées.

10. Instrument chirurgical selon la revendication 9, **caractérisé en ce que** les portions d'extrémité distales (26, 28 ; 26', 28' ; 26", 28") comportent une portion oblique obtenue par découpage ou polissage.

11. Instrument chirurgical selon la revendication 10, **caractérisé en ce qu'**une portion d'extrémité distale respective (26, 28 ; 26', 28' ; 26", 28") présente une surface polie ou coupée oblique (34, 36 ; 34', 36' ; 34", 36") qui s'étend en formant un angle aigu par rapport à un axe médian de la portion d'extrémité distale respective (26, 28 ; 26', 28' ; 26", 28").

12. Instrument chirurgical selon la revendication 10 ou 11, **caractérisé en ce qu'**une surface polie respective (34, 36 ; 34', 36'; 34", 36") ou une surface coupée respective sont disposées de façon à pointer vers l'intérieur lorsque le fil (14 ; 14' ; 60) déplaçable longitudinalement est rétracté dans la lumière (16).

13. Instrument chirurgical selon l'une des revendications 1 à 12, **caractérisé en ce que** l'élément déplaçable longitudinalement comporte deux fentes longitudinales qui s'étendent dans des plans longitudinaux différents de façon à obtenir quatre portions d'extrémité distales.

14. Instrument chirurgical selon l'une des revendications 1 à 13, **caractérisé en ce que** l'élément déplaçable longitudinalement (14 ; 14'; 60) est formé par un fil en matériau superélastique, notamment en alliage superélastique tel que du Nitinol.

15. Instrument chirurgical selon l'une des revendications 1 à 14, **caractérisé en ce que** l'élément déplaçable longitudinalement se présente sous la forme d'un implant (60) apte à sortir totalement de la lumière.

16. Instrument chirurgical selon la revendication 15, **caractérisé en ce que** l'instrument chirurgical comporte un fil coulissant (70) qui est guidé de façon déplaçable longitudinalement dans la lumière du côté proximal de l'élément (60) déplaçable longitudinalement et qui est conformé de telle sorte que l'élément (60) déplaçable longitudinalement, se présentant sous la forme d'un implant, est apte à sortir suffisamment loin de l'orifice distal de la lumière, au moyen du fil coulissant (70), pour que l'implant puisse s'accrocher suffisamment dans le tissu du corps à marquer de sorte que l'implant reste dans le tissu du corps lorsque l'aiguille creuse est rétractée avec le fil coulissant.

17. Instrument chirurgical selon l'une des revendications 1 à 16, **caractérisé en ce qu'**un élément de préhension est fixé à une extrémité proximale de l'aiguille creuse (12), lequel élément de préhension comporte un élément coulissant qui est relié de façon amovible à l'élément déplaçable longitudinalement et qui est mobile suivant un mouvement alternatif entre deux positions longitudinales dont une première position longitudinale correspond à un état de l'élément chirurgical dans lequel les portions d'extrémité distales (26, 28 ; 26', 28') ou au moins une portion la plus courte (26) parmi les portions d'extrémité distales sont totalement rétractées dans la lumière (16) de l'aiguille creuse (12) tandis qu'une deuxième position longitudinale correspond à un état de l'élément chirurgical dans lequel au moins les portions longitudinales incurvées des portions d'extrémité distales (26, 28 ; 26', 28') font saillie de l'orifice ouvert à l'extrémité distale de la lumière (16).

18. Instrument chirurgical selon la revendication 17, **caractérisé en ce que** l'élément coulissant est mobile jusque dans une troisième position dans laquelle l'élément coulissant et donc l'élément de préhension sont détachés de l'élément (14 ; 14') déplaçable longitudinalement.

19. Instrument chirurgical selon l'une des revendications 1 à 18, **caractérisé en ce que** l'élément déplaçable longitudinalement est entouré par une gaine qui est déplaçable longitudinalement par rapport à l'élément déplaçable longitudinalement et qui est apte à sortir de la lumière (16) de l'aiguille creuse (12) conjointement avec l'élément déplaçable longitudinalement.

20. Instrument chirurgical selon la revendication 19, **caractérisé en ce que** la gaine est en matière plastique.
